(19) Europäisches Patentamt
European Patent Office
Office européen des brevets

(11) **EP 2 550 908 A1**

(12) **EUROPEAN PATENT APPLICATION**

(43) Date of publication:
**30.01.2013 Bulletin 2013/05**

(51) Int Cl.:
**A61B 1/00** (2006.01)    **A61B 1/005** (2006.01)

(21) Application number: **11175764.7**

(22) Date of filing: **28.07.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Fraunhofer-Gesellschaft zur Förderung der angewandten Forschung e.V.**
**80686 München (DE)**
• **Friedrich-Alexander-Universität Erlangen-Nürnberg**
**91054 Erlangen (DE)**

(72) Inventors:
• **Wittenberg, Thomas**
**91054 Erlangen (DE)**
• **Münzenmayer, Christian**
**90409 Nürnberg (DE)**

• **Boronat, Javier Gutierrez**
**91088 Bubenreut (DE)**
• **Jahn, Jasper**
**90489 Nürnberg (DE)**
• **Rulsch, Martin**
**91054 Erlangen (DE)**
• **Winter, Christian**
**31141 Hildesheim (DE)**
• **Arzt, Christian**
**91052 Erlangen (DE)**
• **Bergen, Tobias**
**91054 Erlangen (DE)**

(74) Representative: **Schenk, Markus et al**
**Schoppe, Zimmermann, Stöckeler & Zinkler**
**Patentanwälte**
**Postfach 246**
**82043 Pullach bei München (DE)**

(54) **Apparatus for determining a spatial path of a flexible or semi-rigid elongated body**

(57) Embodiments of the present invention provide an apparatus with a flexible or semi-rigid elongated body and a plurality of sensor devices. The plurality of sensor devices are arranged at or in the flexible or semi-rigid elongated body in a manner distributed along a longitudinal direction of the flexible or semi-rigid elongated body. Each sensor device of the plurality of sensor devices is configured to provide information on a local orientation, position and/or curvature of the flexible or semi-rigid elongated body.

FIG 3

EP 2 550 908 A1

## Description

Embodiments of the present invention relate to an apparatus for determining a spatial path of a flexible or semi-rigid elongated body. Some embodiments of the present invention relate to a method and a system for determining an orientation, location, relative position, and/or course of a flexible or semi-rigid longitudinal object such as an endoscope or catheter.

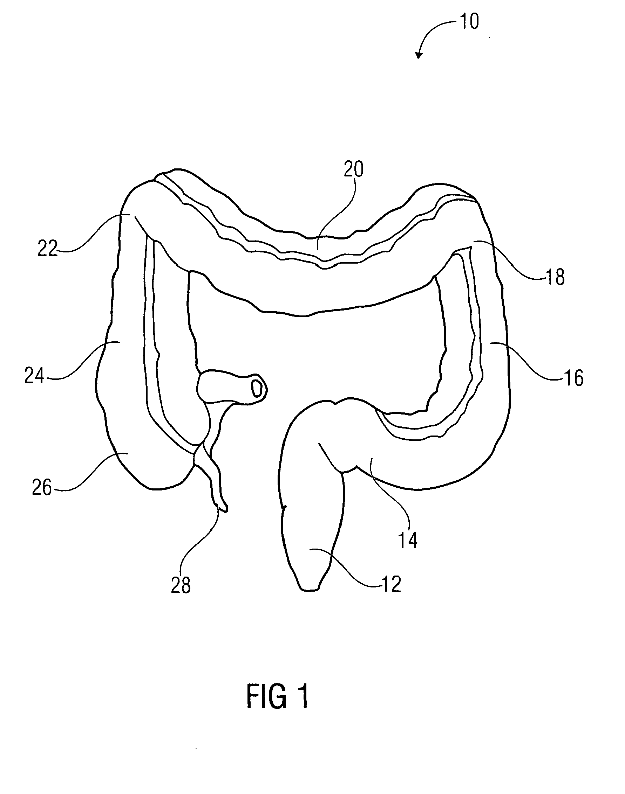

Fig. 1 shows a schematic view of a colon and its sections. The sections of the colon 10 are the rectum 12, colon sigmoldeum 14, colon descendens 16, left flexure 18, colon transversum 20, right flexure 22, colon ascendens 24, cecum 26 and appendix 28. The colonoscopy being currently the best approved precaution for an early detection of the colorectal carcinoma is a technically sophisticated method. The so-called "mirror-advancing", i.e. feeding the flexible video endoscope via the movable colon which is bended several times, is not immediately successful with each patient, as the formation of loops in particular in the so-called sigma (S-bend of the colon or large intestine in the lower abdomen) has to be eliminated and the endoscope has to be correspondingly straightened. This process of mirror-advancing is time-consuming, requires high concentration of the examiner and his assistant and is also sometimes painful for the patient when loops have formed. In particular without a direct or indirect possibility of a visual control of the instrument, the examiner may only divine the actual course and the bending of the endoscope (with respect to the position and orientation) or feel them through the abdominal wall and accordingly has to experiment with the available possibilities of control (push, pull and rotate the endoscope around its own axis, bending the tip in two orientations). Several factors are responsible for the time required for the forward motion to the so-called cecum 26 (the short part of the colon 10 between the appendix 28 and the ileocecal valve, also called cecum 26). These are, apart from the state of the intestine and the amount of liquid still to be sucked off, the formation of loops and the fixing of such loops caused by previous operations. In particular, this formation of loops of the different colon sections (colon sigmuidum 14, left flexure 18, colon transversum 20, right flexure 22, mobile colon ascendens 24, see Fig. 1) leads to an increased time requirement with respect to feeding forward, as the loops have to be "straightened" by corresponding maneuvers of the colonoscope in order to be able to continue the examination fast and with little discomfort for the patient. Apart from this, such loops are also one of the main reasons for not reaching the cecum 26.

As already mentioned, due to the flexible design of a colonoscope, loops can be easily formed during examination of the large intestine. This can result in acute or subsequent pain for the patient and can impair the doctor's ability to orient the colonoscope during the examination. Visualization of the endoscope path can help alleviate these problems.

Guided endoscopy is supposed to shorten the insertion time, to support easy and safe loop elimination and therefore increase the patient's comfort.

A visual support of the endoscopy for position control and correction in the process of mirror-advancing may be executed by a radiological screening, but is always connected with a corresponding radiation exposure.

The ScopeGuide from Olympus (http://www.olympus-europa.com/endoscopy/ 431_ScopeGuide.htm) displays the shape and position of the colonoscope in real time throughout the examination. It's based on magnetic fields generated by small coils built inside the endoscope and picked up by the antenna of ScopeGuide's main unit. From this data a computer reconstructs and displays a schematic picture of the endoscope. The magnetic field generator as well as the computer and display component are combined to a mobile station for easy handling in and between examination rooms.

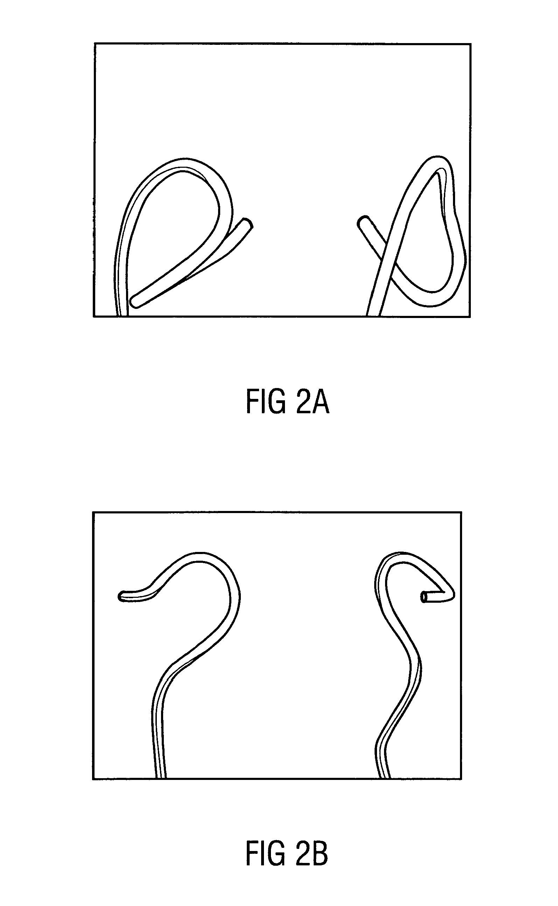

In other words, the known ScopeGuide system of the company Olympus provides a virtual, three-dimensional real time representation of the colonoscope during the complete operation. Along the insertion section (approximately 150 cm flexible shaft) of the endoscope, more than one dozen miniature transmitters (coils) are built in. Each of these transmitters generates an extremely weak electromagnetic field which is received by an external antenna system and evaluated by software. The exact position of each individual transmission coil can be calculated from the respective amplitudes and run-time differences of the different transmit signals. On the basis of the calculated positions, a virtual three-dimensional visualization of the endoscope introduced into the intestine can be represented in real time on a screen (http://www.endoskopie-hamburg.de /scopeguide.htm). The course of the insertion of the endoscope into the large intestine is here indicated simultaneously by a split-screen mode from different directions like for example lateral view or dorsal-ventral view as shown in Figs. 2a and 2b, respectively (http://www.krankenpflege-journal.com/inneremedizin/endoskopie/1673-mirko-feuringvorsorgekoloskopie-neue-endoskope-versprechen-weniger-schmerzen-und-mehr-nutzen.html).

The ScopeGuide system of the company Olympus has been known since approx. 2001, but has only been used in Europe for a few years (approximately since 2006) in few countries. However, in particular for England and Scandinavia such navigation and orientation supporting systems are mandatory for tender.

One disadvantage of this system is the necessity of an antenna which is relatively large as compared to the operating room which is required for receiving the signals and has to be located in the direct vicinity of the patient. This antenna is accordingly either mounted to the endoscopic tower which is placed next to the patient during recording or is mounted or integrated at or in the examination/operation table.

Moreover, in the articles "Endoscopic Orientation Correction" from Kurt Höller, Jochen Penne, Armin Schneider, Jasper Jahn, Javier Guttierrez, Thomas Wittenberg, Hubertus Feussner, and Joachim Hornegger; "Suppression of Shock Based Errors with Gravity Related Endoscopic Image Rectification" from Kurt Höller, Jochen Penne, Armin Schneider, Jasper Jahn, Hani Girgis, Javier Guttierrez, Thomas Wittenberg, Hubertus Feussner and Joachim Hornegger; "Taking Endoscopy to a Higher Dimension: Computer Aided 3-D NOTES" from Kurt Höller, M. Petrunina, Jochen Penne, Armin Schneider, Dirk Wilhelm, Hubertus Feußner and Joachim Hornegger; "Clinical evaluation of Endorientation: Gravity Related Rectification for Endoscopic Images" from Kurt Höller, Armin Schneider, Jasper Jahn, Javier Guttierrez, Thomas Wittenberg, Joachim Hornegger and Hubertus Feussner; "Endoscopic Image Rectification Using Gravity" from Kurt Höller, Armin Schneider, Jasper Jahn, Javier Guttierrez, Thomas Wittenberg, Hubertus Feussner and Joachim Hornegger; "Orientation of Endoscopic Images: Rectification by Gravity" from Kurt Höller, Armin Schneider, Jasper Jahn, Javier Gutierrez, Thomas Wittenberg, Alexander Meining, Stefan von Delius, Joachim Hornegger and Hubertus Feußner; and "An Economical Method for Colonoscope Tracking" from Ching L.Y., Möller K. and Suthakorn J. a project of the University of Erlangen-Nürnberg together with the TU Munich and the Fraunhofer IIS of a flexible video endoscopy system for the colonoscopy or gastroscopy is described, wherein at its tip one single miniaturized acceleration sensor (inertial sensor) in MEMS design (MEMS= Micro-Electro-Mechanical Systems) is mounted or integrated. This sensor provides, in real time (approx. 200 times per second), a vector of three acceleration values (in all three axes) from which directly the position (orientation) of the endoscopy tip can be determined in two degrees of freedom (DoF). As for the orientation determination gravitation is used, there is a singularity, i.e. when the (optical axis of the) endoscopy camera is aligned vertically just as the gravitational vector. At this point, the orientation cannot be determined based on the gravitational vector. Based on a one-time calibration of the external orientation and the continuous information on the relative twist of the endoscopy tip, automatically a so-called artificial horizon can be generated. I.e., no matter from which perspective and orientation an object or cavity is regarded, and independent of how the endoscope is rotated or twisted, the recorded endoscopic image is always corrected by a counter rotation such that on the screen with respect to gravitation "top" is represented as "top" and "bottom" is shown as "bottom". A similar system for evaluating and rendering the endoscopic image data stream using a single inertial sensor was, among others, also presented by the Hanover Medical School. An approach for motion tracking of the endoscopy tip for navigated endoscopy by means of an EM tracker, e.g. using the AURORA system, was presented by Cheng et al. (2010).

Such systems currently are in pre-clinical studies and thus ought to help surgeons active in gastroenterology when using video endoscopes to find their way in the abdomen despite the twisting of the endoscopy tip.

Disadvantages of those systems with respect to the question for determining the orientation, position, location and shape of an endoscope are the purely singular measurements of the orientation or rotation in one location (as described above). Apart from this, a determination of the position, course and bendings of the endoscope is not possible by one single measurement.

For detecting movements at the Fraunhofer IIS a sensor network was developed whose sensor nodes contain triaxial acceleration sensors in MEMS design. With each individual node, as with the above-described "EndOrientation" project, the orientation of each node may be determined in real time. Also by using this system rotations around the gravitational vector may not be observed. One disadvantage of the system thus is that the reconstruction of the orientation is limited to two of three degrees of freedom.

By the combination of acceleration sensors with magnetic field sensors and gyroscopes into an inertial measurement unit (IMU) all three degrees of freedom of the orientation may be determined. One example is the product MTi of the company Xsens. Nevertheless, integrating such sensors and developing a method for determining a orientation, position and location based on the knowledge regarding the orientation of the IMU is very difficult.

Therefore, it is the object of the present invention to provide a concept for sensing a position and/or orientation of a flexible or semi-rigid elongated body that reduces or avoids the above mentioned disadvantages.

This object is solved by an apparatus according to claim 1, a method according to claim 14 and a computer program according to claim 15.

Embodiments of the present invention provide an apparatus with a flexible or semi-rigid elongated body and a plurality of sensor devices. The plurality of sensor devices are arranged at or in the flexible or semi-rigid elongated body in a manner distributed along a longitudinal direction of the flexible or semi-rigid elongated body. Each sensor device of the plurality of sensor devices is configured to provide an information on a local orientation, position and/or curvature of the flexible or semi-rigid elongated body.

Embodiments of the present invention are described herein making reference to the appended drawings.

Fig. 1          shows an illustrative view of a colon and its sections.

Figs. 2a, 2b    show two different views of a visualization of an endoscope generated with the known ScopeGuide System from Olympus.

Fig. 3          shows a schematic view of an apparatus with a flexible or semi-rigid elongated body and a plurality of sensor devices according to an embodiment of the present invention.

Fig. 4          shows a schematic view of a reconstructed path of the endoscope based on the information provided by the sensor nodes.

Fig. 5          shows a schematic representation of an algorithm for reconstructing the path of the endoscope according to an embodiment of the present invention.

Fig. 6          shows a diagram of the effects of the measurement precision on the reconstructed path of the endoscope.

Fig. 7          shows a diagram of the error propagation effected by three segments of the endoscope in an exaggerated size.

Fig. 8          shows a schematic view of the sensor network according to an embodiment of the present invention.

Fig.9          shows a schematic view of a single sensor node according to an embodiment of the present invention.

Fig. 10          shows a schematic view of a single sensor node according to an alternative embodiment of the present invention.

Equal or equivalent elements or elements with equal or equivalent functionality are denoted in the following description by equal or equivalent reference numerals.

In the following description, a plurality of details are set forth to provide a more thorough explanation of embodiments of the present invention. However, it will be apparent to one skilled in the art that embodiments of the present invention may be practiced without these specific details. In other instances, well-known structures and devices are shown in block diagram form rather than in detail in order to avoid obscuring embodiments of the present invention. In addition, features of the different embodiments described hereinafter may be combined with each other, unless specifically noted otherwise.

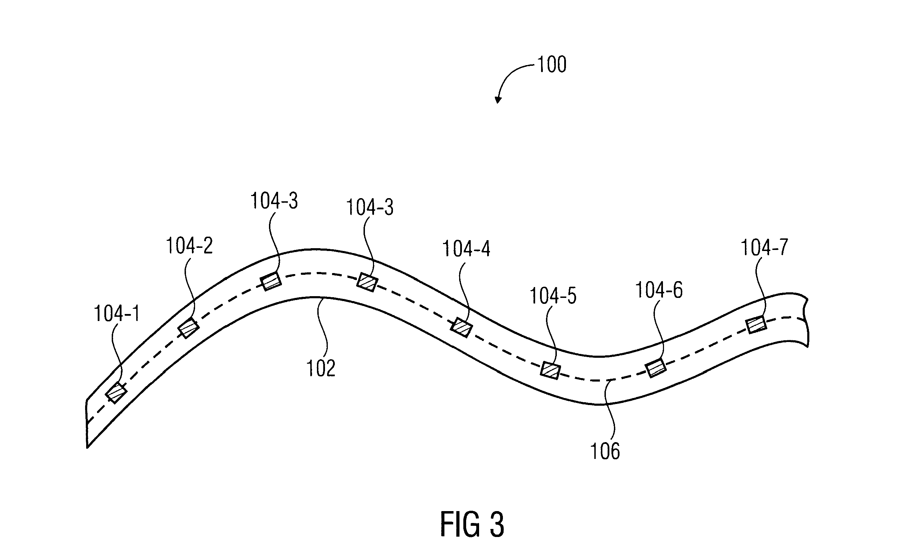

Fig. 3 shows a schematic view of an apparatus 100 with a flexible or semi-rigid elongated body 102 (or longitudinal object) and a plurality of sensor devices 104_1 to 104_n (n = 7) according to embodiments of the present invention. The plurality of sensor devices 104_1 to 104_n (n = 7) are arranged at or in the flexible or semi-rigid elongated body 102 in a manner distributed along a longitudinal direction of the flexible or semi-rigid elongated body 102. Each sensor device of the plurality of sensor devices 104_1 to 104_n (n = 7) is configured to provide an information on a local orientation, position and/or curvature of the flexible or semi-rigid elongated body 102.

The apparatus 100 shown in Fig. 1 exemplarily comprises seven sensors 104_1 to 104_n (n = 7). Naturally, the apparatus 100 can comprise up to n sensors 104_1 to 104_n, wherein n is a natural number greater than or equal to two (n ≥ 2).

In some embodiments, the apparatus 100 can further comprise an evaluator configured to evaluate the information provided by each sensor device in order to determine a spatial path 106 along which the flexible or semi-rigid elongated body 102 is bended/folded.

Furthermore, the plurality of sensor devices 104_1 to 104_n can be arranged at predefined distances to each other along the flexible or semi-rigid elongated body 102, wherein the evaluator can be configured to determine the spatial path 106 along which the flexible or semi-rigid elongated body 102 is bended/folded based on the predefined distances.

Moreover, the evaluator can be configured to provide an information describing the spatial path 106 along which the flexible or semi-rigid elongated body 102 is bended/folded, wherein the apparatus 100 can further comprise a monitor configured to visualize the spatial path 106 along which the flexible or semi-rigid elongated body 102 is bended/folded based on the information provided by the evaluator.

In embodiments, each sensor device of the plurality of sensor devices 104_1 to 104_n can comprise at least one of an acceleration-, inertial-, magnetic-field-, optical- or strain-stress-sensor. Moreover, a sensor device can be referred to as sensor group in the case that the sensor device comprises more than one sensor, e.g. an acceleration- and magnetic-field-sensor.

For example, each sensor device of plurality of sensor devices 104_1 to 104_n can comprise optical components. The pinhole camera principle could be used for optical measurement of the spatial bending angle. This involves directing a light source at a plate with a hole a few millimeters away to reduce the light down to a point and projecting it onto an optically sensitive detector field an identical distance farther away. If this arrangement is subjected to a bend or curve, the position of the light spot on the detector field changes. If this change is brought into relation with the bending angle, such an arrangement can be used for measurement of the spatial curvature. CCD sensors could be suitable as a detector field, and these are already commercially available in sizes significantly below 2x2 cm$^2$ (sensor area).

Alternatively, each sensor device of the plurality of sensor devices 104_1 to 104_n can comprise a resistance strain gauges. The strain of an object can be measured by a resistance strain gauge. The resistance in a conductive film changes based on its elongation or stretching. The measurement value, called the "gauge factor", can be related to the force applied. Two resistance strain gauges offset by 90° around the length axis of the flexible or semi-rigid elongated body 102 could supply relatively robust measurement values for this sensor point.

Optionally, each sensor device of the plurality of sensor devices 104_1 to 104_n can comprise a sensor for inductively measuring the length of the flexible or semi-rigid elongated body 102. A resonant oscillator circuit reacts extremely sensitively to changes in the electromagnetic properties of the inductor environment without the need for an external electromagnetic influence. This sensitivity can be utilized by calibrating and measuring the elongations of a length-variable inductor. Multiple such miniaturized inductors could be combined into a spatial bending sensor.

Furthermore, each sensor device of the plurality of sensor devices 104_1 to 104_n can comprise a gyroscope. A gyroscope is capable of precisely measuring small, even jerky movements. However, the measurement value "drifts" off after a relatively short time even though the sensor is at rest. The magnetic-field-sensor could be used as a locally stable reference signal as long as the gyroscope (possibly in combination with the acceleration-sensor) does not detect any change. In the event of a movement, the algorithm may rely more heavily on the sensors that are independent of the magnetic field.

The apparatus 100 according to the concept of the present invention allows to detect the orientation, curvature, position (e.g. relative or absolute position) and/or course of a flexible or semi-rigid elongated body 102 (e.g. a flexible endoscope, tube or cable) of any length and in real time. In addition, the detected orientation, curvature, position and/or course of the flexible or semi-rigid elongated body can be visualized on a monitor, e.g. in several perspectives or views.

For example, the flexible or semi-rigid elongated body 102 can be a flexible video endoscope 102 (or colonoscope) for examining the large intestine, where the apparatus 100 can be used to control and visualize the orientation, curvature, (relative) position and/or course of the endoscope 102 during mirror-advancing interactively. Thereby, a formation of loops of the endoscope 102 and thus pain for the patient can be reduced or even avoided. In addition, error sources and necessary time for examination can be reduced.

Likewise, the plurality of sensor devices 104_1 to 104_n (e.g. each sensor device having an inertial sensor and a magnetic field sensor) can be arranged at or in a semi-rigid cable 102 for industrial applications which can be brushed through empty tubes. Thereby, a course (and, if applicable, a formation of loops) of the semi-rigid cable 102 can be controlled and visualized using the plurality of sensor devices 104_1 to 104_n and related algorithms, which can be performed, for example, by the evaluator.

The concept of the present invention is based on the plurality of sensor devices 104_1 to 104_n, or in other words, on an array of sensor nodes or sensor groups that are configured to sense an orientational bending angle at the respective nodes. For example, each sensor group can comprise a combination of one or several inertial sensors (e.g. acceleration and/or rotational rate sensors, the latter also designated as gyroscopes, e.g. in MEMS technology) together with one or several (three-dimensional) magnetic field sensors (or a combination of one-dimensional sensors). Alternatively, an optical detector according to the principle of a pin-hole camera can be used that is configured to detect a direct bending by evaluating the light or laser point coming in through the pin-hole on a course sensor area. Optionally, a pressure-strain-sensor on the basis of a polarization sensor can be used.

In some embodiments, the plurality of sensor devices 104_1 to 104_n can be networked by means of a network (e.g. a serial or parallel network), wherein the evaluator can be coupled to the network by means of a network controller. Due to the arrangement of the plurality of sensor devices 104_1 to 104_n at or in the flexible or semi-rigid elongated body 102 in a manner distributed along a longitudinal direction of the flexible or semi-rigid elongated body 102 and the common evaluation of the information (e.g. sensor data) provided by each sensor device, the plurality of sensor devices 104_1 to 104_n can be referred to as sensor network. Furthermore, each sensor device of the plurality of sensor devices 104_1 to 104_n can be referred to as sensor node.

The plurality of sensor devices 104_1 to 104_n can be integrated or mounted at or in the flexible or semi-rigid elongated body 102 in defined and known positions with defined and known (not necessarily equal distance) distances, e.g. at or in the supporting body.

The information, e.g. a sensor signal, provided by each sensor device of the plurality of sensor devices 104_1 to 104_n (or sensor group of the plurality of sensor groups 104_1 to 104_n) can be detected and calculated by the evaluator. In some embodiments, the evaluator can be a (personal) computer, e.g. having a program code for performing a method described herein.

Moreover, the evaluator can be configured to determine spatial sub-paths between the plurality of sensor devices 104_1 to 104_n (or segments) based on the information provided by each sensor device, and to determine the spatial path 106 along which the flexible or semi-rigid elongated body is bended/folded based on the sub-paths between the plurality of sensor devices 104_1 to 104_n. For example, based on the known distances of the plurality of sensor devices 104_1 to 104_n with respect to each other and the information provided by each sensor device, e.g. raw data (or manipulated raw data in the form of rotation, speed orientation or location or a further combination of the raw data) determined separately by each sensor device of the plurality of sensor devices 104_1 to 104_n and by mathematical models for imaging the mechanical setup and the measured variables, shifting, position, curvature, orientation and location of a sub-path between two sensor devices of the plurality of sensor devices 104_1 to 104_n always can be calculated. Based on the determined parameters, the sub-paths may be rendered graphically and be represented on a monitor in several views, such as front, top or side.

Each sensor device of the plurality of sensor devices 104_1 to 104_n (or sensor group of the plurality of sensor groups 104_1 to 104_n) can comprise a combination of an acceleration- and magnetic-field-sensor. In addition, each sensor device of the plurality of sensor devices 104_1 to 104_n can comprise a rotational-rate-sensor (gyroscope). The acceleration-sensor can be used to detect the gravitation and thus rotations around axes perpendicular to a gravitational vector. Moreover, the azimuth can be determined with respect to a global coordinate system from a terrestrial magnetic field measurements or its projection onto a horizontal plane. Hence, two independent supplementing measured variables are available to determine the orientation of each sensor device of the plurality of sensor devices 104_1 to 104_n unequivocally. However, interferences acting on the sensors may have to be modeled and intercepted by algorithms, which can be performed, for example, by the evaluator, in order to subtract distortions of the information, e.g. measurement data or sensor data, provided by each sensor device of the plurality of sensor devices 104_1 to 104_n.

In the following, embodiments of the present invention are described, where the flexible or semi-rigid elongated body 102 is an endoscope. Naturally, the following description is also applicable to the flexible or semi-rigid elongated body shown in Fig. 1.

In some embodiments, the apparatus 100 can further comprise a camera arranged at or in a tip of the endoscope 102, wherein the evaluator can be configured to determine the spatial path 106 along which the endoscope 102 is bended/folded further based on the information provided by the camera. In other words, in addition to the information (e.g. measured data or sensor data) provided by each sensor device of the plurality of sensor devices 104_1 to 104_n, a graphical or pictorial information provided by the camera at the tip of the endoscope 102 can be used in order to determine the orientation, position, location, curvature and shape of the endoscope 102. From the information provided by the camera, e.g. a recorded video data stream, the motion of the camera tip can be approximated with methods for image processing. Based on a relative movement between two or more recorded images of the video data stream, an image-to-image transformation can be estimated that allows to map subsequent pictures. The estimation of the image-to-image transformation can be performed, for example, by the method of optical flow or by tracking relevant image features (feature tracking). On the basis of the image-to-image transformation, a relative movement (i.e., rotation and/or transformation) of the camera can be calculated. This process can be similar to methods used in navigation, guidance and robotics, lidar-methods or TOF cameras (TOF = Time of Flight) as described in the articles "Kamaragestütztes inertiales Navigationssystem mit Sensorfusion" from the Fraunhofer Technologie-Entwicklungsgruppe; "GNSS-independent Navigation Solution Using Integrated LiDAR Data" from Susca Sara; "A Novel Approach for 3-D Video-Endoscopy" from Penne, J., Jesorsky, O., Winter, M., Horbach, T., Krüger, S., Hohenberger, W. and Hornegger, J.; and "Time-of-Flight 3-D Endoscopy" from Jochen Penne, Kurt Höller, Michael Stürmer, Thomas Schrauder, Armin Schneider, Rainer Engelbrecht, Huber Feußner, Bernhard Schmauss and Joachim Hornegger.

By a suitable calibration, the visually detected parameters or the parameters derived there from can be combined with the parameters derived from the information (e.g. measured data) provided by each sensor device of the plurality of sensor devices 104_1 to 104_n.

The evaluator can be configured to provide the information describing the spatial path 106 along which the endoscope 102 is bended/folded such that the spatial path 106 along which the endoscope 102 is bended/folded is visualized on the monitor in a 3D (three-dimensional) manner. In other words, the visualization of the endoscope 102 based on the information provided by each sensor device of the plurality of sensor devices 104_1 to 104_n can be performed as a three-dimensional visualization on a digital monitor. Alternatively, the visualization of the endoscope 102 can be performed on a three-dimensional monitor. In both cases, a decisive improvement is the visualization of the path 106 within the regular endoscope 102 image, i.e. as an overlay or lateral fade-in in the image (picture-in-picture).

A substantial information for the examiner is that it becomes clear which part of the endoscope 102 lays in front (i.e., closer to the viewer) and/or back in order to be able to avoid a formation of loops. This can be achieved by using an additional color coding in the visualization (e.g. green coloring of the endoscope part which is closer to the viewer, red coloring for the covered part). Therefore, the evaluator can be configured to provide the information describing the spatial path 106 along which the endoscope 102 is bended/folded such that the spatial path 106 along which the endoscope 102 is bended/folded is visualized on the monitor using a plurality of colors, each color of the plurality of colors representing a depth layer in a three-dimensional space.

The evaluator can be configured to provide the information describing the spatial path 106 along which the endoscope 102 is bended/folded such that the spatial path 106 along which the endoscope 102 is bended/folded that is visualized on the monitor corresponds to at least one of a plurality of switchable perspectives. In other words, any number of (in practice, e.g. two or three) standard perspectives (e.g. cranial-caudal, dorsal-ventral, lateral) of the three-dimensional visualization of the endoscope 102 can be defined which can be set manually once and then be selected, e.g. by control keys, footswitches, voice control etc.

Furthermore, the evaluator can be configured to provide the information describing the spatial path 106 along which the endoscope 102 is bended/folded such that the perspective of the spatial path 106 along which the endoscope 102 is bended/folded is visualized on the monitor based on the orientation of the endoscope 102 with respect to a reference coordinate system. In other words, an automatic switching of the perspective can be implemented for the visualization depending on the position of the patient (e.g. prone, supply, lateral).

The automatic switching of the perspective can be calculated by an overall evaluation of the information provided by the plurality of sensor devices 104_1 to 104_n, in particular of the information describing a gravitation value or parameter. Alternatively, a separate sensor that can be coupled to the patient (e.g. by means of a belt, adhesive technology, integrative in shirt/textile) can be used to determine the gravitational direction, where the position of the patient can be derived there from.

The apparatus 100 according to the concept of the present invention does not require an antenna nor a dedicated and/or generated electro-magnetic field in order to determine the spatial path 106 along which the flexible or semi-rigid elongated body 102 is bended/folded. In addition, a standard perspective for the visualization can be detected automatically from the position of the patient and can be switched if required. Furthermore, the visualization of the endoscope path 106 can be improved by means of a picture-in-picture visualization.

Furthermore, a relative position of a flexible or semi-rigid elongated body (e.g. a endoscope, colonoscope, rigid cable or semi-rigid tube) including its twists and/or loops can be detected by means of the plurality of sensor devices 104_1 to 104_n, and be visualized in several perspectives (e.g. "front", "side" and "top") on a monitor in real-time.

To determine the spatial orientation at each sensor node, two sensors can be installed in each sensor node with complementary measurement ranges for avoiding undefined position information. The magnetic field sensor (MF) measures the strength of the terrestrial magnetic field while the acceleration sensor (ACC) measures the acceleration due to gravity acting on the sensor node. Via control electronics (e.g. network controller), a connected evaluator, e.g. a PC, communicates with the sensor nodes 104_1 to 104_n and receives the information, e.g. measurement data or sensor data, provided by the sensor nodes 104_1 to 104_n at regular intervals. Software, which can be performed, e.g. by the evaluator, merges the information, e.g. sensor data, provided by the sensor nodes 104_1 to 104_n and visualizes the path 106 of the endoscope 102 based on the analyzed sensor data.

As already mentioned, loops can easily be formed during examination of the large intestine by colonoscope (flexible endoscope). This can result in acute or subsequent pain for the patient and can impair the doctor's ability to orient the colonoscope during the examination. In order to counteract these problems, the apparatus 100 according to the present invention allows to detect and suitably visualize such loops. Thereby, the orientation of the endoscope 102 can be determined at defined points (sensor nodes 104_1 to 104_n) and the position along the entire endoscope 102 can be calculated on this basis and displayed on the monitor or screen.

The apparatus 100 does not require a dedicated and/or generated electromagnetic field in order to determine the path 106 of the endoscope 102. Rather, natural earth-magnetic and gravitational fields can be used for measuring the orientation or path of the endoscope 102. The determination of direction can be performed by a sensor network with sensor nodes 104_1 to 104_n comprising magnetic field and acceleration sensors situated at specific intervals along the entire length of the endoscope 102.

In theory, orientation of a static or slow-moving rigid body, like the endoscope 102, can be determined from the measured gravity and local magnetic field vectors. The orientation of the body can be described in an earth-fixed coordinate system $X_e Y_e Z_e$. The body coordinate system $X_b Y_b Z_b$ can be attached to the body or endoscope 102 whose orientation is to be measured. The sensor module or sensor node can have its own coordinate system $X_s Y_s Z_s$ corresponding to the axes of the accelerometer/magnetometer. For simplification, it can be assumed that the sensor coordinate system coincides with the body coordinate system.

The sensor node can be in its reference orientation when its axes are aligned with those of the earth coordinate system. To estimate the actual orientation of the sensor node, the rotation about the y-axis (pitch angle) and the rotation about the x-axis (roll angle) can be calculated from the acceleration vector.

The calculated angles can be used to align the X- and Y-axes of the magnetic field measurement vector to the earth coordinate system. The deviation between the rotated measurement vector and the local reference magnetic field can be used to calculate the rotation of the sensor about the Z-axis (heading angle).

The sensor nodes can be individual circuit boards having all relevant components for data collection, communication and interfaces. These circuit boards can be attached, e.g. at 3 cm, 4 cm, 5 cm, 6 cm, 7 cm, 10 cm, 20 cm, 30 cm 50 cm or 100 cm intervals, along the flexible portion of the endoscope 102 adhesively or mechanically (e.g. by mechanical clamps or) such that the circuit board move according to the local orientation of the endoscope 102 at the respective positions.

The sensor network can be connected to the evaluator, e.g. to a PC via USB (USB = Universal Serial Bus). When new measurement data is available, the sensor network sends this to the PC. The software framework, which can be performed, e.g. by the evaluator, can use this measurement data to calculate the orientation for each sensor node 104_1 to 104_n: yaw, pitch and roll angle. The known orientation of the sensor nodes 104_1 to 104_n and a known spacing between the sensor nodes are the basis for reconstructing the endoscope 102 path 106.

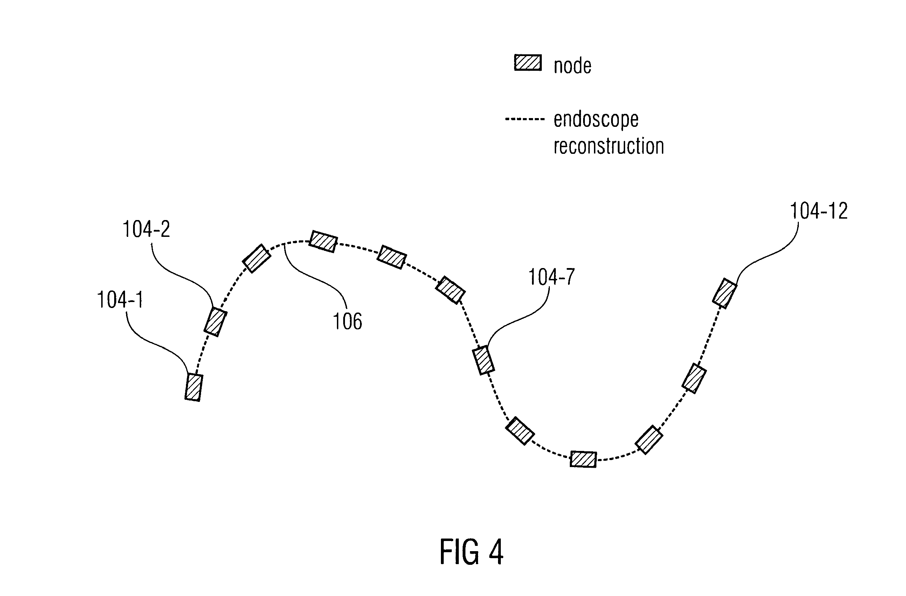

Fig. 4 shows a schematic view of a reconstructed path 106 of the endoscope 102 based on the information, e.g. orientation, provided by the sensor nodes 104_1 to 104_n (n = 12). Thereby, every sensor node 104_1 to 104_n (n = 12) represents a node for a spline interpolation. The resulting spline is an estimate of the path 106.

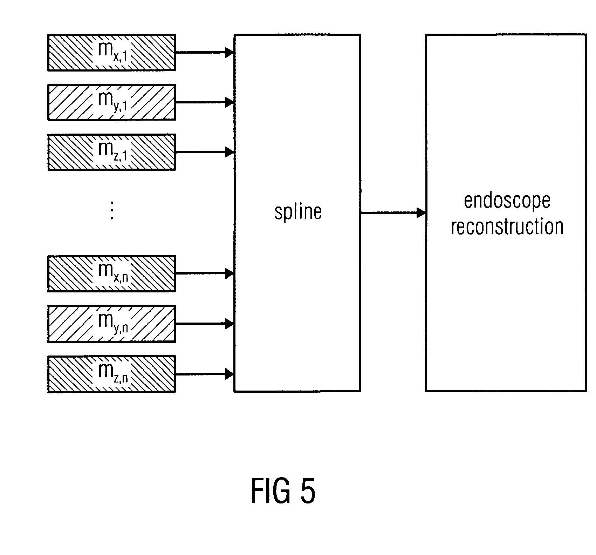

To reconstruct the endoscope 102 path 106, the progress of the endoscope 102 in the X, Y and Z directions ($m_x, m_y, m_z$) can be calculated based on the yaw and pitch angle. Starting from the first sensor node 104_1, a polynomial with a length equal to the distance between neighboring sensor nodes can be calculated for each neighboring pair of sensor nodes 104_1 to 104_n (n = 12), taking into consideration the orientation of the sensor nodes 104_1 to 104_n (n = 12). The final reconstruction of the endoscope 102 path 106 arises from a combination of the individual polynomials as shown in Fig. 5.

A theoretical average measurement precision of each sensor node 104_1 to 104_n (n = 12) can be defined in horizontal and vertical direction.

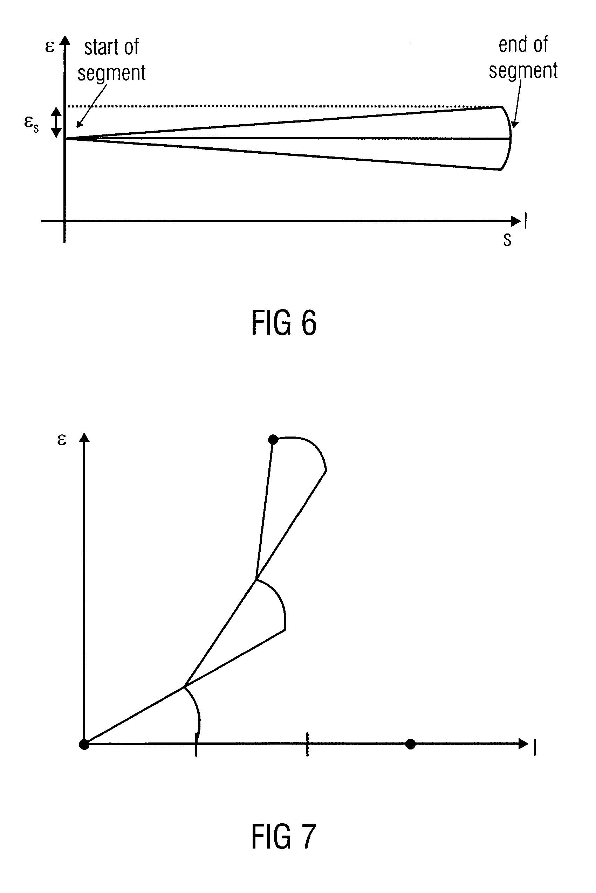

Fig. 6 shows a diagram of the effects of the measurement precision on the reconstructed path 106 of the endoscope 102. In other words, Fig. 6 schematically represents the margin of uncertainty. Thereby, the ordinate describes the deviation $\varepsilon$, where the abscissa describes the length 1 of the segment. With segment length s, $\varepsilon_s$ is the possible deviation between the actual and reconstructed end of the segment.

Fig. 7 shows a diagram of the error propagation effected by three segments of the endoscope in an exaggerated size. Thereby, the ordinate describes the deviation $\varepsilon$, where the abscissa describes the length 1 of the segments. The more segments a loop encompasses, the more imprecise the loop detection can become due to the possibility of error propagation. The possible error after the first segment can persist through to the end.

After the second segment, an additional possible error can be added, which also can persist to the end of the chain, etc. Generalized for n segments, this means that the first error can effect the position determination ($\varepsilon_1$) n times, the second error (n-1),etc.:

$$\varepsilon_l = \varepsilon_s \sum_{i=1}^{n} i = \varepsilon_s \frac{n(n+1)}{2}$$

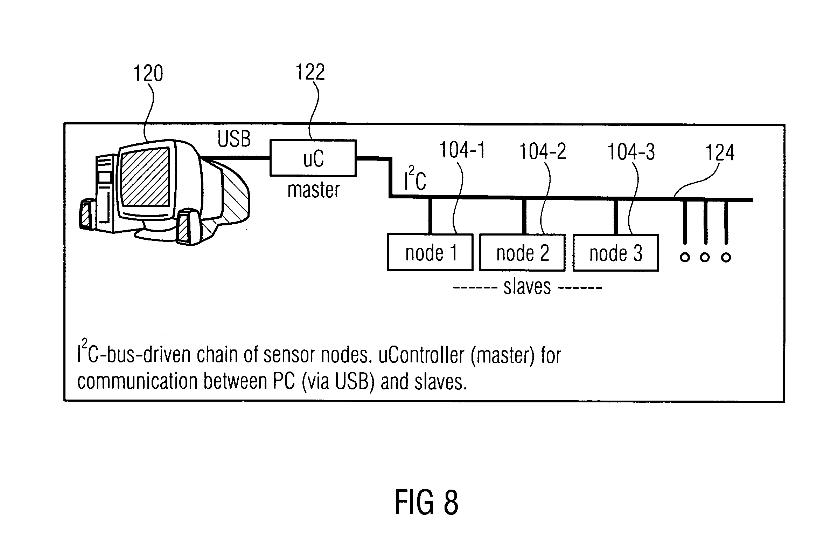

Fig. 8 shows a schematic view of the sensor network according to an embodiment of the present invention. The sensor nodes 104_1 to 104_n (n = 12) can be networked by means of a network 124, wherein the evaluator 120 can be coupled to the network 124 by means of a network controller 122. As shown in Fig. 8, the network 124 can be a I²C bus (I²C = Inter-Integrated Circuit), where the evaluator 120 can be a PC, and where the network controller 122 can be a microcontroller that is connected to the PC, e.g. via USB. The sensor network (or sensor chain) for determining the endoscope 102 path 106 is based on individual sensor nodes 104_1 to 104_n (n = 12). All sensor nodes 104_1 to 104_n (n = 12) (slaves) can be connected via a shared I²C bus 124 (two power supply and two data lines) to a microcontroller 122 (master), which queries and controls the sensor nodes (see Fig. 8). The microcontroller 122 is connected to a PC 120 via USB.

Because the individual sensor nodes 104_1 to 104_n (n = 12) are not able to measure their absolute position but only their orientation with respect to the gravitational or terrestrial magnetic field, they can be affixed in the endoscope 102 (or construction channel) at defined intervals. The path of the endoscope 102 can be reconstructed based on the known spacing and the measured orientations.

Every sensor node 104_1 to 104_n (n = 12) can comprise one magnetic-field-sensor (MF) and one acceleration-sensor (ACC) (or accelerometer). In case that at least one of these components cannot be uniquely addressed, the communication with each sensor node 104_1 to 104_n (n = 12) can be realized via an electronic circuit for address decoding (FPGA). Thereby, the FPGA (FPGA = Field-Programmable-Gate-Array) can be included in the magnetic-field-sensor or integrated as a separate component.

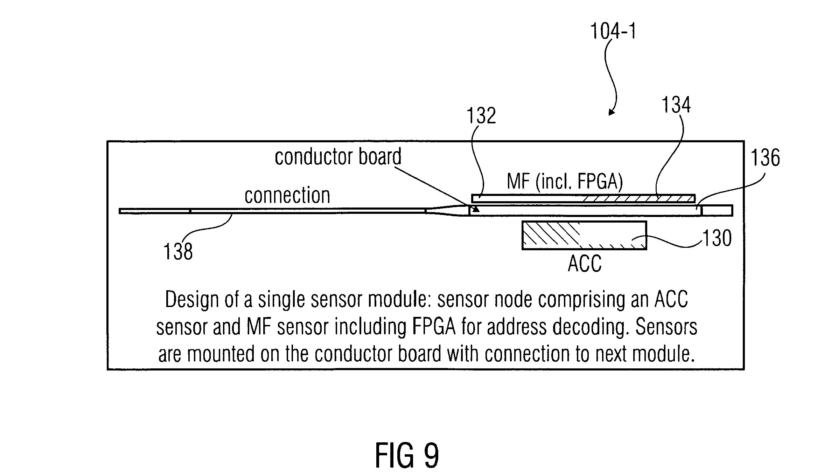

Fig. 9 shows a schematic view of a single sensor node (e.g. 104_1) according to an embodiment of the present invention. The sensor node comprises (e.g. 104_1) an acceleration-sensor 130 (ACC), a magnetic field sensor 132 including the FPGA 134, a conductor board 136 and a connection 138.

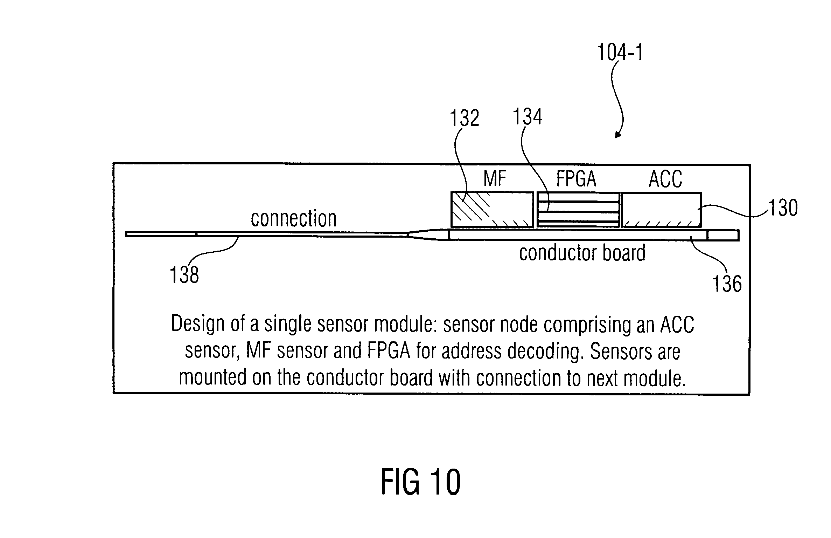

Fig. 10 shows a schematic view of a single sensor node (e.g. 104_1) according to an alternative embodiment of the present invention. The sensor node comprises (e.g. 104_1) an acceleration-sensor 130 (ACC), a magnetic field sensor 132, a separate FPGA 134, a conductor board 136 and a connection 138.

In Figs. 9 and 10, the term sensor module refers to a sensor node including the required connecting elements. Because it is very difficult to manufacture the entire sensor chain as a single component, a design with individual sensor modules can be used. In this case, it is possible to flexibly connect practically any number of sensor modules. Because the system should be permanently installed in the endoscope, all components should permit a certain bending radius and also withstand the cleaning temperature.

Every sensor node 104_1 to 104_n (n = 12) can be addressed and queried by a microcontroller 122 e.g.

situated outside of the construction channel. The measurements of the individual sensor nodes 104_1 to 104_n (n = 12) can be performed in parallel, independently of each other. Referring to Fig. 9, the sensor nodes 104_1 to 104_n (n = 12) can be controlled by the electronic circuit of the MF-ASIC (ASIC = Application-Specific Integrated Circuit). Referring to Fig. 10, an additional FPGA can be required. In both cases, these control electronics can implement an SPI interface (SPI = Serial Peripheral Interface) by which the associated ACC sensor can be controlled. The microcontroller 122 (master) can query the data of the MF and ACC sensors via the $I^2C$ interface.

If no measurement is performed at a sensor node 104_1 to 104_n (n = 12), both sensors can be in a power-down-mode. When the available measurement values are queried, a new measurement can be performed immediately and the sensor then switches to the power-down-mode to keep the power dissipation and interference fields due to current in the supply wires as low as possible. To obtain the data from all sensor nodes 104_1 to 104_n (n = 12) (full measurement), the PC 120 activates the microcontroller 122 over the USB interface, and the microcontroller 122 in turn queries the measurement values of all sensor nodes 104_1 to 104_n (n = 12) in sequence and then passes them to the PC 120 for processing and evaluation.

Further embodiments of the present invention provide a method comprising providing an information on a local orientation, position and/or curvature of a flexible or semi-rigid elongated body 102 with each of a plurality of sensor devices 104_1 to 104_n, wherein the plurality of sensor devices 104_1 to 104_n are arranged at or in the flexible or semi-rigid elongated body 102 in a manner distributed along a longitudinal direction of the flexible or semi-rigid elongated body 102.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus. Some or all of the method steps may be executed by (or using) a hardware apparatus, like for example, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a digital storage medium, for example a floppy disk, a DVD, a Blu-Ray, a CD, a ROM, a PROM, an EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may for example be stored on a machine readable carrier.

Other embodiments comprise the computer program for performing one of the methods described herein, stored on a machine readable carrier.

In other words, an embodiment of the inventive method is, therefore, a computer program having a program code for performing one of the methods described herein, when the computer program runs on a computer.

A further embodiment of the inventive methods is, therefore, a data carrier (or a digital storage medium, or a computer-readable medium) comprising, recorded thereon, the computer program for performing one of the methods described herein. The data carrier, the digital storage medium or the recorded medium are typically tangible and/or non-transitionary.

A further embodiment of the inventive method is, therefore, a data stream or a sequence of signals representing the computer program for performing one of the methods described herein. The data stream or the sequence of signals may for example be configured to be transferred via a data communication connection, for example via the Internet.

A further embodiment comprises a processing means, for example a computer, or a programmable logic device, configured to or adapted to perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

The above described embodiments are merely illustrative for the principles of the present invention. It is understood that modifications and variations of the arrangements and the details described herein will be apparent to others skilled in the art. It is the intent, therefore, to be limited only by the scope of the impending patent claims and not by the specific details presented by way of description and explanation of the embodiments herein.

**Claims**

1. Apparatus (100), comprising:

   a flexible or semi-rigid elongated body (102); and
   a plurality of sensor devices (104_1 to 104_n) arranged at or in the flexible or semi-rigid elongated body (102) in a manner distributed along a longitudinal direction of the flexible or semi-rigid elongated body (102);

   wherein each sensor device of the plurality of sensor devices (104_1 to 104_n) is configured to provide an information on a local orientation, position and/or curvature of the flexible or semi-rigid elongated body (102).

2. Apparatus (100) according to claim 1, further comprising an evaluator configured to evaluate the information provided by each sensor device in order to determine an spatial path (106) along which the flexible or semi-rigid elongated body (102) is bended/folded.

3. Apparatus (100) according to claim 2, wherein the plurality of sensor devices (104_1 to 104_n) are arranged at predefined distances to each other along the flexible or semi-rigid elongated body (102), wherein the evaluator is configured to determine the spatial path (106) along which the flexible or semi-rigid elongated body (102) is bended/folded based on the predefined distances.

4. Apparatus (100) according to claim 2 or 3, wherein the evaluator is configured to determine spatial sub-paths between the plurality of sensor devices (104_1 to 104_n) based on the information provided by each sensor device, and to determine the spatial path (106) along which the flexible or semi-rigid elongated body is bended/folded based on the sub-paths between the plurality of sensor devices (104_1 to 104_n).

5. Apparatus (100) according to one of the claims 1 to 4, wherein the evaluator is configured to provide an information describing the spatial path (106) along which the flexible or semi-rigid elongated body (102) is bended/folded, wherein the apparatus (100) further comprises a monitor configured to visualize the spatial path (106) along which the flexible or semi-rigid elongated body (102) is bended/folded based on the information provided by the evaluator.

6. Apparatus (100) according to claim 5, wherein the evaluator is configured to provide the information describing the spatial path (106) along which the flexible or semi-rigid elongated body (102) is bended/folded such that the spatial path (106) along which the flexible or semi-rigid elongated body (102) is bended/folded is showed on the monitor such that the determined information in all three dimensions is visualized.

7. Apparatus (100) according to claim 5, wherein the evaluator is configured to provide the information describing the spatial path (106) along which the flexible or semi-rigid elongated body (102) is bended/folded such that the spatial path (106) along which the flexible or semi-rigid elongated body (102) is bended/folded is visualized on the monitor using a plurality of colors, each color of the plurality of colors representing a depth layer in three-dimensional space.

8. Apparatus (100) according to claim 5, wherein the evaluator is configured to provide the information describing the spatial path (106) along which the flexible or semi-rigid elongated body (102) is bended/folded such that the spatial path (106) along which the flexible or semi-rigid elongated body (102) is bended/folded that is visualized on the monitor corresponds to at least one of a plurality of switchable perspectives.

9. Apparatus (100) according to claim 8, wherein the evaluator is configured to provide the information describing the spatial path (106) along which the flexible or semi-rigid elongated body (102) is bended/folded such that the perspective of the spatial path (106) along which the flexible or semi-rigid elongated body (102) is bended/folded visualized on the monitor is based on the orientation of the flexible or semi-rigid elongated body (102) with respect to a reference coordinate system.

10. Apparatus (100) to one of the claims 1 to 9, wherein each sensor device of the plurality of sensor devices (104_1 to 104_n) comprises at least one of an acceleration-, inertial-, magnetic-field-, optical- or strain-stress-sensor.

11. Apparatus (100) according to one of the claims 1 to 10, wherein the plurality of sensor devices (104_1 to 104_n) are networked by means of a network, wherein the evaluator is coupled to the network by means of a network controller.

**12.** Apparatus (100) according to one of the claims 1 to 11, further comprising a camera arranged at or in a tip of the flexible or semi-rigid elongated body (102), wherein the evaluator is configured to determine the spatial path (106) along which the flexible or semi-rigid elongated body (102) is bended/folded further based on the information and/or processed information provided by the camera.

**13.** Apparatus (100) to one of the claims 1 to 12, wherein the flexible or semi-rigid elongated body (102) is a coloscope, endoscope, cable or tube.

**14.** Method, comprising;
providing an information on a local orientation, position and/or curvature of a flexible or semi-rigid elongated body (102) with each of a plurality of sensor devices (104_1 to 104_n);
wherein the plurality of sensor devices (104_1 to 104_n) are arranged at or in the flexible or semi-rigid elongated body (102) in a manner distributed along a longitudinal direction of the flexible or semi-rigid elongated body (102).

**15.** Computer program having a program code for performing, when running on a computer, field programmable gate array, digital signal processor or microprocessor, a method according to claim 14.

FIG 1

FIG 2A

FIG 2B

FIG 3

FIG 4

FIG 5

FIG 6

FIG 7

120    122
USB

uC
master

I²C    104-1    104-2    104-3    124

node 1    node 2    node 3    o o o

------ slaves ------

I²C-bus-driven chain of sensor nodes. uController (master) for communication between PC (via USB) and slaves.

FIG 8

EP 2 550 908 A1

104-1

132    134

136

conductor board

MF (incl. FPGA)

connection

138    130

ACC

Design of a single sensor module: sensor node comprising an ACC sensor and MF sensor including FPGA for address decoding. Sensors are mounted on the conductor board with connection to next module.

FIG 9

Design of a single sensor module: sensor node comprising an ACC sensor, MF sensor and FPGA for address decoding. Sensors are mounted on the conductor board with connection to next module.

FIG 10

Europäisches
Patentamt

European
Patent Office

Office européen
des brevets

# EUROPEAN SEARCH REPORT

**Application Number**

EP 11 17 5764

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEE YIK CHING ET AL: "Non-radiological colonoscope tracking image guided colonoscopy using commercially available electromagnetic tracking system", ROBOTICS AUTOMATION AND MECHATRONICS (RAM), 2010 IEEE CONFERENCE ON, IEEE, PISCATAWAY, NJ, USA, 28 June 2010 (2010-06-28), pages 62-67, XP031710266, ISBN: 978-1-4244-6503-3 * abstract * * section I-V * * figures 1-11 * | 1-15 | INV.<br>A61B1/00<br><br>ADD.<br>A61B1/005 |
| X | EP 1 857 038 A2 (ETHICON ENDO SURGERY INC [US]) 21 November 2007 (2007-11-21) * abstract * * paragraph [0004] * * paragraph [0030] - paragraph [0036] * * figures 1-8 * | 1,10, 13-15 | |

**TECHNICAL FIELDS SEARCHED (IPC)**

A61B

The present search report has been drawn up for all claims

| Place of search | Date of completion of the search | Examiner |
|---|---|---|
| The Hague | 25 January 2012 | Marteau, Frédéric |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 17 5764

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2012

| Patent document cited in search report | | Publication date | Patent family member(s) | | Publication date |
|---|---|---|---|---|---|
| EP 1857038 | A2 | 21-11-2007 | AU | 2007202209 A1 | 06-12-2007 |
| | | | CA | 2589149 A1 | 18-11-2007 |
| | | | CN | 101088451 A | 19-12-2007 |
| | | | EP | 1857038 A2 | 21-11-2007 |
| | | | JP | 2007319668 A | 13-12-2007 |
| | | | US | 2007270649 A1 | 22-11-2007 |

EPO FORM P0459

For more details about this annex : see Official Journal of the European Patent Office, No. 12/82